Europäisches Patentamt

European Patent Office

Office européen des brevets

(11) Publication number: **0 129 197**
**B1**

## EUROPEAN PATENT SPECIFICATION

(45) Date of publication of patent specification: **18.10.89**

(51) Int. Cl.⁴: **A 61 K 7/06**

(21) Application number: **84106744.0**

(22) Date of filing: **13.06.84**

(54) **Hair growth promoting composition.**

(30) Priority: **20.06.83 JP 110436/83**
**13.01.84 JP 4568/84**

(43) Date of publication of application:
**27.12.84 Bulletin 84/52**

(45) Publication of the grant of the patent:
**18.10.89 Bulletin 89/42**

(84) Designated Contracting States:
**AT CH DE FR GB LI**

(56) References cited:
**BE-A- 868 440**
**DE-A-1 492 030**
**DE-A-2 240 187**
**FR-A- 379 368**
**FR-A- 732 935**
**GB-A- 824 353**

(73) Proprietor: **Kao Corporation**
**14-10, Nihonbashi Kayabacho 1-chome**
**Chuo-Ku Tokyo 103 (JP)**

(72) Inventor: **Yamamoto, Hiromi**
**6-23-18, Nakashizu**
**Sakura-shi Chiba-ken (JP)**
Inventor: **Okumura, Takeo**
**2-7-7, Takinoi**
**Funabashi-shi Chiba-ken (JP)**
Inventor: **Eguchi, Yasuteru**
**513-6, Minemachi**
**Utsunomiya-shi Tochigi-ken (JP)**
Inventor: **Hattori, Michihiro**
**6-5-1-603, Motoimaizumi**
**Utsunomiya-shi Tochigi-ken (JP)**

(74) Representative: **Wächtershäuser, Günter, Dr.**
**Tal 29**
**D-8000 München 2 (DE)**

**Description**

i) Field of the Invention:
This invention relates to hair growth promoting compositions and more particularly, to hair growth promoting compositions comprising blood flow increasing agents and/or hair matrix cell activators, and carbon dioxide gas.

ii) Description of the Prior Art:
In conventional hair growth promoting compositions, it is usual to use synthetic substances or extracts from natural products having the blood flow increasing action or hair matrix cell activating action. However, satisfactory effects cannot be obtained when such substances or extracts are used only in small amounts. Although certain effects are obtained when large amounts of the substances or extracts are used, they tend to objectionably irritate sites on which the growth promoting composition is applied. If such growth promoting composition is continuously applied, dermatitis may appear.

Meanwhile, it is known that carbon dioxide has the vasodilative action. A carbon dioxide bath is clinically used for rehabilitation. Carbon dioxide has been also used in hair growth compositions for a long time. See FR—A—379,368, FR—A—732,935 or DE—A—1,492,030 for instance.

Summary of the Invention
With the above in mind, we have made extensive studies to suitably apply carbon dioxide to hair growth promoting compositions. As a result, it was found that use of carbon dioxide in combination with blood flow increasing agents and/or hair matrix cell activators is very effective. More particularly, it was found that when carbon dioxide is used in combination with blood flow increasing agents, it is possible to increase the flow of blood through scalp and retain the increase of the blood flow. Likewise, the combination of carbon dioxide with hair matrix cell activators results in vasilidation of capillary vessels in the scalp, with the hair matrix cell activator efficiently arriving at the matrix cells and serving to activate the cells. Thus, these combinations lead to reduction of falling-out of the hair and acceleration of growth of the hair. The present invention is accomplished based on the above finding.

According to the present invention, there is provided a hair growth promoting composition which comprises a blood flow increasing agent and/or a hair matrix cell activator in admixture with carbon dioxide gas, and tightly sealed in a pressure-resistant container.

Detailed Description of the Invention and Preferred Embodiments
The blood flow increasing agents to be used in the present invention include, for example, synthetic substances or substances derived from natural products such as dl-α-tocopheryl acetate, pyridoxine dicaprylate, biotin, nicotinic acid and derivatives thereof, tolazoline hydrochloride, glyceryl glycyrrhetinate, hinokitiol, and extracts from various plants such as Swertia japonica, garlic, *Angelica acutiloba* Kitagawa, *Cnidium,* dried orange peel, camomile, and peppermint. Of these, dl-α-tocopheryl acetate, and *Swertia japonica, Angelica acutiloba* Kitagawa, and *Cnidium* extracts are preferred.

The hair matrix cell activators include, for example, synthetic or natural products such as vitamin A acid, pantothenic acid and derivatives thereof, pantothenyl alcohol and derivatives thereof, l-aspartic acid and derivatives thereof, photosensitive principle No. 301, ethynylestradiol, placenta extract and iron chlorophyll. Of these, vitamin A acid, potassium pantothenate, pantothenyl alcohol, and placenta extract are preferred.

These blood flow increasing agents and/or hair matrix cell activators are ordinarily used by dissolving in water or mixtures of water and lower alcohols. Preferably, the blood flow increasing agent and/or hair matrix cell activator is used in an amount of 0.001 to 5 wt% (hereinafter referred to simply as %) of the hair growth promoting composition. Smaller amounts are not favorable because satisfactory effects cannot be obtained, whereas larger amounts result in disagreeable stimulation against the scalp.

Carbon dioxide is known to be present as $CO_2$ molecules when dissolved in an acidic solution and show the vasodilative action. In this sense, the hair growth promoting composition of the present invention is generally adjusted to be in the range of between pH 7 and 4.5, preferably from 4.5 to 6.5. It will be noted that the hair growth promoting composition of the invention becomes more acidic when carbon dioxide is charged under pressure and dissolved in the growth promoting composition. The final pH range should be adjusted as defined above. For the adjustment of the pH, there are used organic acids such as citric acid, tartaric acid and lactic acid or salts thereof, and phosphoric acid or salts thereof.

For the incorporation of carbon dioxide into the hair growth promoting composition, several methods are conveniently used. In one such method, a hair growth promoting composition is placed in a pressure-resistant container, into which high pressure gas is introduced. Another method comprises placing a hair growth promoting composition containing a carbonate such as sodium hydrogencarbonate, to which is added a pH adjuster thereby generating carbon and the container is immediately sealed tightly. A further method makes use of dry ice pellets for generating carbon dioxide. Of these, the method using high pressure gas is preferred.

By this, part of carbon dioxide is dissolved in the hair growth promoting composition and part thereof is present as a gas in the container. In the practice of the invention, it is important that carbon dioxide be dissolved and incorporated in the hair growth promoting composition. The amount of the incorporated

2

carbon dioxide is preferably over 60 ppm. Less amounts are unfavorable because satisfactory vasodilative action cannot be expected. The amount of carbon dioxide may be controlled depending on the charge of incorporated carbon dioxide (charged under pressure). In general, it is preferred that the pressure in the container is in the range of 1.2 to 8 kg/cm$^2$·G at a temperature of 35°C.

The pressure-resistant container used in the practice of the invention should be a container which can withstand the above-defined pressure for a period of from tight sealing till use in order to keep the hair growth promoting composition in a sealed condition. Examples of such container include metal containers such as aluminium and tin, resin containers such as acetal resins and polycarbonate resins, and glass containers.

The container has a discharge nozzle. If a diameter of the discharge nozzle is small, the filling liquid is sprayed as mist, whereas if the diameter is large, the liquid is sprayed as foams or droplets. As a matter of course, the spraying state may differ depending on the inner pressure of the container. With known aerosol sprayers, the discharge nozzle thereof has a diameter of below 0.3 mm. If such a discharge nozzle is applied to the hair growth promoting composition of the present invention, satisfactory results cannot be obtained because the filling liquid is sprayed in the form of mist and carbon dioxide is immediately lost from the liquid. To avoid this, it is necessary to keep a long residence time of carbon dioxide in hair growth promoting composition. In this sense, it is preferable to apply the composition directly on the scalp in the form of foams or droplets while permitting the hair growth promoting composition to be deposited on the hair only in amounts as small as possible. Upon the application, it becomes possible to generate carbon dioxide on the scalp. For the purposes, the discharge nozzle is preferred to have a diameter of 0.3 to 1.5 mm and a length of 1 to 15 cm. In order to prevent reduction in amount of dissolved carbon dioxide and maintain a constant spraying condition, it is favorable to use an aerosol injector as disclosed in Japanese Laid-open Application No. 57—153752 in which a miniature bomb of carbon dioxide is built in a container, by which when the pressure of carbon dioxide lowers, fresh carbon dioxide can be supplied to keep a concentration of carbon dioxide constant from an initial stage of use till completion.

Beside the above-described essential ingredients, the hair growth promoting composition of the invention may further comprise other ingredients used in ordinary cosmetics such as oil substrates, emollients, gelling agents, various emulsifiers, perfumes, preservatives such as parahydroxybenzoates, antioxidants such as butyl hydroxyanisole, colorants such as dyes, refrigerants such as menthol, bactericides and humectants such as propylene glycol.

As described above, the hair growth promoting composition of the present invention which comprises blood flow increasing agents and/or hair matrix cell activators, and carbon dioxide serves to increase the blood flow within a short time and retain the increase of the blood flow to an extent. The blood flow increasing agents and/or hair matrix cell activators can be efficiently arrived at matrix cells of the hair. As a result, good effect of preventing falling-out of hair is attained. Use of carbon dioxide enables stimulative blood flow increasing agents and/or hair matrix cell activators to be reduced in amounts, leading to the possibility of continuous application of the hair growth promoting composition with the expectation of more improved effects. Moreover, upon gasification of carbon dioxide dissolved in the hair growth promoting composition, agreeable stimulation and a kind of massage are given to the scalp. Because the hair growth promoting composition itself is weakly acidic and is thus close to the human skin in pH, favorable influences on the skin can be expected.

The present invention is particularly illustrated by way of examples.

Reference 1

The hair of each of C3H mice (male) on the back skin at the resting stage thereof was cut so that no damage was given to the back skin. The state of growth of the hair at a subsequent growing stage was observed. Sites where the growth of the hair on the skin surface was observed were checked as a function of time. A ratio, r, of an area of the sites to an area of the cut portion was calculated. The results are shown in Table 1.

## Table 1

| | r* (%) | | | | | | | | |
|---|---|---|---|---|---|---|---|---|---|
| Number of Days after Hair Cutting (Days)<br><br>Animal | 6 | 8 | 10 | 12 | 14 | 16 | 20 | 35 | 45 |
| 20 days old mice | 0 | 100 | 100 | 100 | 100 | 100 | 100 | 100 | 100 |
| 65 days old mice | 0 | 0 | 12 | 53 | 84 | 100 | 100 | 100 | 100 |
| 188 days old mice | 0 | 0 | 2 | 17 | 30 | 42 | 65 | 95 | 98 |
| 314 days old mice | 0 | 0 | 0 | 0 | 7 | 14 | 18 | 52 | 53 |

$$* \quad \frac{\text{Area of Hair Growth at Growing Stage}}{\text{Hair-cut Area}} \times 100 (\%)$$

As will be seen from Table 1, the area of sites at which the commencement of growth of the hair delays increases with age.

Example 1

The hair of each of 108 days old C3H mice (male) on the skin of the back at the resting stage was cut without damaging the skin. The mice were divided into 7 groups and applied on the hair-cut portion thereof with hair growth promoting compositions indicated in Table 2 every day. The growth of the hair on the back of each mouse was observed. An area of sites where the growth of the hair was recognized on the skin surface was measured, and its ratio, r, to an area of the cut portion was calculated. The results are shown in Table 3.

[Compositions]

Table 2

| Ingredients | Inventive Compositions | | Comparative Compositions | | | | |
|---|---|---|---|---|---|---|---|
| | (1) | (2) | (1) | (2) | (3) | (4) | (5) |
| **Composition (%)** | | | | | | | |
| Carbon dioxide | 1.00 | 1.00 | - | - | - | - | - |
| dℓ-α-Tocopheryl acetate | 1.00 | - | - | - | 1.00 | - | - |
| Japanese chirate (*Swertia Japonica*) extract | 1.00 | 1.00 | 2.00 | - | - | - | - |
| Garlic extract | - | - | - | 1.00 | - | - | - |
| Hinokitiol | - | - | - | - | - | 0.10 | - |
| Glyceryl glycyrrhetinate | - | - | - | - | - | 0.05 | - |
| Physiological saline solution | - | - | - | - | - | - | balance |
| Lactic acid | 0.133 | 0.133 | 0.133 | 0.133 | 0.133 | 0.133 | 0.133 |
| Sodium lactate | 0.3 | 0.3 | 0.3 | 0.3 | 0.3 | 0.3 | 0.3 |
| 50 v/v% Ethanol aqueous solution | balance | balance | balance | balance | balance | balance | - |

EP 0 129 197 B1

[Results]

Table 3

| Hair Growth Promoting Composition | Number of Days after Hair Cutting (Days) | r* (%) | | | | |
|---|---|---|---|---|---|---|
| | | 9 | 10 | 11 | 12 | 13 |
| Composition of Invention (1) | | 5 | 72 | 93 | 98 | 100 |
| " (2) | | 1 | 67 | 90 | 97 | 100 |
| Comparative Composition (1) | | 0 | 52 | 82 | 90 | 95 |
| " (2) | | 0 | 42 | 77 | 89 | 93 |
| " (3) | | 0 | 38 | 64 | 87 | 92 |
| " (4) | | 0 | 37 | 59 | 78 | 88 |
| " (5) | | 0 | 17 | 38 | 52 | 58 |

As will be seen from Table 3, the hair growth promoting compositions of the invention comprising carbon dioxide are effective in facilitating commencement of the growth of the hair.

Example 2

Hair growth promoting compositions indicated in Table 4 were prepared to determine their effect in the same manner as in Example 1. The results are shown in Table 5.

[Composition]

EP 0 129 197 B1

Table 4

| Ingredients | Composition of Invention | | | | Comparative Composition | | | | |
|---|---|---|---|---|---|---|---|---|---|
| | (3) | (4) | (5) | (6) | (6) | (7) | (8) | (9) | (10) |
| **Composition (%)** | | | | | | | | | |
| Carbon dioxide | 1.00 | 1.00 | 1.00 | 1.00 | – | – | – | – | – |
| Vitamin A acid | – | – | – | 1.00 | – | – | – | – | – |
| Potassium pantothenate | 1.00 | – | – | – | – | – | 1.00 | – | – |
| Pantothenyl alcohol | – | 1.00 | – | – | 1.00 | – | – | – | – |
| Placenta extract | – | – | 1.00 | – | – | 1.00 | – | – | – |
| Hinokitiol | – | – | – | – | – | – | – | 0.10 | – |
| Glyceryl glycyrrhetinate | – | – | – | – | – | – | – | 0.05 | – |
| Physiological saline solution | – | – | – | – | – | – | – | – | balance |
| Lactic acid | 0.133 | 0.133 | 0.133 | 0.133 | 0.133 | 0.133 | 0.133 | 0.133 | 0.133 |
| Sodium lactate | 0.3 | 0.3 | 0.3 | 0.3 | 0.3 | 0.3 | 0.3 | 0.3 | 0.3 |
| 50 v/v% Ethanol aqueous solution | balance | balance | balance | balance | balance | balance | balance | balance | balance |

# EP 0 129 197 B1

[Results]

## Table 5

| Hair Growth Promoting Composition \ Number of Days after Hair Cutting (Days) | r* (%) | | | | |
|---|---|---|---|---|---|
| | 9 | 10 | 11 | 12 | 13 |
| Composition of Invention (3) | 3 | 60 | 95 | 99 | 100 |
| " (4) | 3 | 65 | 96 | 98 | 100 |
| " (5) | 2 | 68 | 89 | 95 | 100 |
| " (6) | 3 | 66 | 93 | 98 | 100 |
| Comparative Composition (6) | 0 | 51 | 79 | 85 | 92 |
| " (7) | 0 | 50 | 77 | 82 | 90 |
| " (8) | 0 | 35 | 66 | 79 | 88 |
| " (9) | 0 | 37 | 59 | 78 | 88 |
| " (10) | 0 | 17 | 38 | 52 | 58 |

* Same as indicated in Table 1.

As will be seen from Table 5, the hair growth

promoting compositions of the invention comprising

As will be seen from Table 5, the hair growth promoting compositions of the invention comprising carbon dioxide are effective in facilitating commencement of the growth of the hair.

## Reference 2

Thirty to forty year old men who are considered to have a genealogical predisposition to male alopecia were subjected to measurement of the periodic distribution of the hair at the top of the head. For comparison, normal men of the same generation and men suffered from the alopecia were similarly measured with regard to the hair periodic distribution. The results are shown in Table 6.

Table 6

| Subject | Number of Subjects | Rate of Hair at Resting Stage* (%) |
|---|---|---|
| Normal men | 20 | 6.4 |
| Male alopecia | 10 | 34.8 |
| Men having the predisposition to male alopecia | 7 | 13.6 |

\* Average value

As will be seen from Table 6, the men who suffer the alopecia and have the predisposition of the alopecia are higher than the normal men with regard to the rate of the hair at the resting stage.

Example 3

Hair growth promoting compositions indicated in Table 7 were continuously used over one year by men who had the predisposition of male alopecia and an increasing rate of the hair at the resting stage and men who were in the progress of the alopecia to check the rate of the hair at the resting stage prior to and after the use. The results are shown in Table 8.

[Composition]

Table 7

| Ingredients | Inventive Composition (A) | Comparative Composition (B) |
|---|---|---|
| Composition (%) | | |
| 95 v/v% Ethanol | 50.00 | 50.00 |
| Glycerine | 1.00 | 1.00 |
| Ethylene oxide-added (n=40) hardened castor oil | 1.00 | 1.00 |
| ℓ-Menthol | 0.20 | 0.20 |
| Perfume | 0.50 | 0.50 |
| Japanese chirate extract | 1.00 | 1.00 |
| d ℓ-α-Tocopheryl acetate | 0.50 | 0.50 |
| Carbon dioxide | 1.50 | – |
| Lactic acid | 0.133 | 0.133 |
| Sodium lactate | 0.3 | 0.3 |
| Purified water | balance | balance |

[Results]

Table 8

| Hair Growth Promoting Composition | Subject | Rate of Hair at Resting Stage (%) | |
| --- | --- | --- | --- |
| | | Prior to Use | After Use |
| Inventive Composition (A) | No. 1 | 15 | 16 |
| | No. 2 | 21 | 18 |
| | No. 3 | 14 | 14 |
| | No. 4 | 18 | 15 |
| Comparative Composition (B) | No. 5 | 22 | 24 |
| | No. 6 | 16 | 17 |
| | No. 7 | 15 | 19 |
| | No. 8 | 18 | 22 |

As will be seen from the results of Table 8, the hair growth promoting composition of the invention comprising carbon dioxide serves to reduce the rate of the hair at the resting stage.

Example 4

The general procedure of example 3 was repeated using hair growth promoting compositons indicated in Table 9. The results are shown in Table 10.

[Composition]

Table 9

| Ingredients | Inventive Composition (C) | Comparative Composition (D) |
|---|---|---|
| **Composition (%)** | | |
| 95 v/v% Ethanol | 50.00 | 50.00 |
| Glycerine | 1.00 | 1.00 |
| Polyoxyethylene(40) hydrogenated castor oil | 1.00 | 1.00 |
| ℓ-Menthol | 0.20 | 0.20 |
| Perfume | 0.50 | 0.50 |
| Potassium pantothenate | 1.00 | 1.00 |
| Carbon dioxide | 1.50 | - |
| Lactic acid | 0.133 | 0.133 |
| Sodium lactate | 0.3 | 0.3 |
| Purified water | balance | balance |

[Results]

Table 10

| Hair Growth Promoting Composition | Subject | Rate of Hair at Resting Stage (%) | |
|---|---|---|---|
| | | Prior to Use | After Use |
| Inventive Composition (C) | No. 9 | 19 | 15 |
| | No. 10 | 20 | 17 |
| | No. 11 | 16 | 17 |
| | No. 12 | 26 | 20 |
| Comparative Composition (D) | No. 13 | 20 | 24 |
| | No. 14 | 15 | 19 |
| | No. 15 | 15 | 15 |
| | No. 16 | 20 | 24 |

As will be seen from the results of Table 10, the hair growth promoting composition of the invention comprising carbon dioxide serves to reduce the rate of the hair at the resting stage.

# EP 0 129 197 B1

**Claims for Contracting States: CH DE FR GB LI**

1. A hair growth promoting composition comprising a solution of a blood flow increasing agent and/or a hair matrix cell activator in admixture with carbon dioxide gas, and tightly sealed in a pressure-resistant container.

2. The hair growth promoting composition according to Claim 1, wherein pH of said hair growth promoting composition is comprised between 7 and 4.5.

3. The hair growth promoting composition according to Claim 1, wherein said blood flow increasing agent is used in a concentration of 0.001 to 5 wt% of the total composition.

4. The hair growth promoting composition according to Claim 1, wherein the carbon dioxide gas is used in an amount not less than 60 ppm.

**Claims for Contracting State: AT**

1. A method of preparing a hair growth promoting composition, which process comprises providing a solution of a blood flow increasing agent and/or a hair matrix cell activator in admixture with carbon dioxide gas and tightly sealing said hair growth promoting composition in a pressure-resistant container.

2. The method according to Claim 1, wherein the pH of said hair growth promoting composition is between 7 and 4.5.

3. The method according to Claim 1, wherein said blood flow increasing agent is used in a concentration of 0.001 to 5 wt.-% of the total composition.

4. The method according to Claim 1, wherein the carbon dioxide gas is used in an amount not less than 60 ppm.

**Patentansprüche für die Vertragsstaaten: CH LI DE FR GB**

1. Haarwuchsstimulierendes Mittel, umfassend eine Lösung eines Mittels zur Förderung der Durchblutung und/oder eines Haarmatrixzellenaktivators im Gemisch mit Kohlendioxidgas und dicht verschlossen in einem druckfesten Behälter.

2. Haarwuchsstimulierendes Mittel gemäß Anspruch 1, wobei der pH-Wert des haaarwuchsstimulierenden Mittels zwischen 7 und 4,5 beträgt.

3. Haarwuchsstimulierendes Mittel gemäß Anspruch 1, wobei das Mittel zur Förderung der Durchblutung in einer Konzentration von 0,001 bis 5 Gew.-% der Gesamtzusammensetzung verwendet wird.

4. Haarwuchsstimulierendes Mittel gemäß Anspruch 1, wobei das Kohlendioxidgas in einer Menge von nicht weniger als 60 TpM verwendet wird.

**Patentansprüche für den Vertragsstaat: AT**

1. Verfahren zur Herstellung eines haarwuchsstimulierenden Mittels, umfassend die Bereitstellung einer Lösung von einem Mittel zur Steigerung der Durchblutung und/oder eines Haarmatrixzellenaktivators im Gemisch mit Kohlendioxidgas und das dichte Verschließen des haarwuchstimulierenden Mittels in einem druckfesten Behälter.

2. Verfahren gemäß Anspruch 1, wobei der pH des haarwuchsstimulierenden Mittels zwischen 7 und 4,5 beträgt.

3. Verfahren gemäß Anspruch 1, wobei das Mittel zur Steigerung der Durchblutung in einer Konzentration von 0,001 bis 5 Gew.-% der Gesamtzusammensetzung verwendet wird.

4. Verfahren gemäß Anspruch 1, wobei das Kohlendioxidgas in einer Menge von nicht weniger als 60 TpM verwendet wird.

**Revendications pour les Etats contractants: CH LI DE FR GB**

1. Composition favorisant la pousse des cheveux comprenant une solution d'un agent augmentant la circulation du sang et/ou d'un activateur des cellules de la matrice des cheveux en mélange avec du dioxyde de carbone gazeux, et scellée de façon étanche dans un récipient résistant à la pression.

2. Composition favorisant la pousse des cheveux selon la revendication 1, dans laquelle le pH de ladite composition favorisant la pousse des cheveux est compris entre 7 et 4,5.

3. Composition favorisant la pousse des cheveux selon la revendication 1, dans laquelle l'agent augmentant la circulation du sang est utilisé dans une concentration de 0,001 à 5% en poids de la composition totale.

4. Composition favorisant la pousse des cheveux selon la revendication 1, dans laquelle le dioxyde de carbone gazeux est utilisé en une quantité non inférieure à 60 ppm.

12

# EP 0 129 197 B1

**Revendications pour l'Etat contractant: AT**

1. Procédé de préparation d'une composition favorisant la pousse des cheveux, ledit procédé consistant à préparer une solution d'une agent augmentant la circulation du sang et/ou d'un activateur des cellules de la matrice des cheveux en mélange avec du dioxyde de carbone gazeux, et à sceller de façon étanche ladite composition favorisant la pousse des cheveux dans un récipient résistant à la pression.

2. Procédé selon la revendication 1, dans lequel le pH de ladite composition favorisant la pousse des cheveux est compris entre 7 et 4,5.

3. Procédé selon la revendication 1, dans lequel ledit agent augmentant la circulation du sang est utilisé dans une concentration de 0,001 à 5% en poids de la composition totale.

4. Procédé selon la revendication 1, dans lequel le dioxyde de carbone gazeux est utilisé en une quantité non inférieure à 60 ppm.